# FASCICULE DE BREVET EUROPEEN

(11) **EP 2 026 835 B1**
(45) Date de publication et mention de la délivrance du brevet: **21.08.2013**
(21) Numéro de dépôt: 07766109.8
(22) Date de dépôt: 29.05.2007
(51) Int. Cl.: A61K 35/08, A61K 38/48, A61K 36/896, A61K 9/00, A61K 31/70, A61K 31/765, A61K 36/16, A61K 36/38, A61K 36/738, A61K 36/8965, A61K 31/685, A61K 36/734

(54) **COMPOSITIONS NASALE ET BUCCALE COMPRENANT UN EXTRAIT DE RUSCUS ACULEATUS DE LA RUTINE, POUR LUTTER CONTRE LE RONFLEMENT**
NASALE UND ORALE ZUSAMMENSETZUNGEN ENTHALTEND RUSCUS ACULEATUS EXTRAKT UND RUTIN, ZUR SCHNARCHREGULIERUNG
NASAL AND BUCCAL COMPOSITIONS COMPRISING A RUSCUS ACULEATUS EXTRACT AND RUTIN, FOR CONTROLLING SNORING

(30) Priorité: 01.06.2006 FR 0651998; 19.02.2007 FR 0701176
(43) Date de publication de la demande: 25.02.2009
(73) Titulaire: Omega Pharma Capital NV, 9810 Nazareth (BE)
(72) Inventeur: ANTON, Jean-Christophe, 67000 Strasbourg (FR); MARIN, Denis, 78870 Bailly (FR); SONIE, Serge, 14600 La-Rivière-Saint-Sauveur (FR)
(74) Mandataire: Jansen, Bart Antonius Johannes
(86) Numéro de dépôt international: PCT/FR2007/051341
(87) Numéro de publication internationale: WO 2007/138224

(56) Documents cités:
- WO-A-2004/000272
- WO-A-2004/037237
- WO-A2-03/070178
- FR-A- 2 876 585
- NONE GIVEN: "Ruscus aculeatus (Butcher's broom)." ALTERNATIVE MEDICINE REVIEW, vol. 6, no. 6, 6 décembre 2001 (2001-12-06), pages 608-612, XP002404230

## Description

La présente invention concerne le domaine du traitement et de la prévention du ronflement et plus particulièrement du ronflement chez certains gros ronfleurs. Chez les gros ronfleurs, un ensemble de phénomènes complexes est mis en jeu qui produit un ronflement caractéristique. Chez ces personnes, l'origine du ronflement est fréquemment pharyngée et également nasale. Or, l'action ciblée mais non simultanée de certains produits sur le pharynx et la luette ne peut résoudre totalement le problème de certains gros ronfleurs. 1l s'agit en effet d'intervenir sur des phénomènes qui impliquent à la fois les structures anatomiques du nez et du pharynx. Une action simultanée sur ces deux cibles peut permettre une meilleure efficacité.

Le problème général du ronflement Environ 42 à 60% des adultes ronflent de temps en temps; 20% des hommes et 10% des femmes ronflent de manière habituelle. Un ronflement moyen atteint facilement un niveau sonore de 45 dB à 60 dB (le bruit d'une voix), tandis qu'un ronflement majeur peut dépasser les 95 dB, ce qui correspond au bruit du passage d'un camion. Chez les gros ronfleurs, les ronflements dépassent souvent les 60 dB et peuvent entraîner des troubles sérieux, non seulement chez le ronfleur lui-même mais aussi chez les personnes qui vivent à ses côtés et ne parviennent pas à dormir à cause du bruit. En effet, chez les gros ronfleurs et leur entourage, le sommeil est souvent désorganisé à cause du bruit et de l'intensité des ronflements. Les causes du ronflement varient d'une personne à 35 l'autre. Au niveau anatomique, le ronflement se produit principalement lorsque les voies respiratoires au niveau de la gorge (pharynx) se rétrécissent après la relaxation des muscles qui normalement maintiennent le passage ouvert. Ce rétrécissement provoque un flux d'air turbulent et des vibrations au niveau du palais mou et à la base de la langue, ce qui crée le son du ronflement. Chez les gros ronfleurs, vient se superposer à un ronflement dont l'origine est le pharynx, un ronflement d'origine nasale.

Les deux causes principales du ronflement d'origine nasale sont une obstruction des voies respiratoires nasales et une déformation du nez. Lors d'une obstruction des voies nasales (nez encombré ou bloqué), le sujet requiert un effort supplémentaire pour inspirer. Cette action peut créer un effet de vide dans la gorge et une modification des passages des flux d'air au niveau de la cavité nasale qui provoquent un ronflement. De plus, l'obstruction des voies nasales impose au sujet de respirer par la bouche et est souvent associée à un ronflement provenant de la gorge.

Ainsi, l'apparition du ronflement chez les gros ronfleurs est due à plusieurs facteurs qui interagissent simultanément les uns avec les autres: principalement des vibrations au niveau du pharynx et un rétrécissement des voies nasales.

### Ronflement au niveau du pharynx

Les vibrations au niveau du pharynx sont la conséquence d'un voile trop long, d'une position d'endormissement en décubitus dorsal et d'un rétrécissement de la cavité buccale. L'existence d'un voile trop long est le facteur le plus facilement compréhensible. Cette hypertrophie du voile atteint relativement peu les muscles de ce dernier, mais touche surtout les tissus graisseux qui les entourent, et la muqueuse. La luette s'allonge, les piliers postérieurs des amygdales s'épaississent et s'élargissent en deux demi-rideaux prolongeant le voile vers le bas. Le deuxième facteur responsable de l'apparition du ronflement est le décubitus dorsal (la position sur le dos du corps endormi). Cette position entraîne le voile détendu à reposer sur la paroi postérieure du pharynx. Dans cette position anatomique, le passage de l'air inspiratoire nasal peut facilement le soulever, le faire flotter et vibrer. Si le dormeur est couché sur le ventre, le voile tombe en avant, et ne risque pas de toucher la paroi postérieure ou même latérale du pharynx ; ainsi dégagé il laisse grand ouvert derrière lui le couloir aérien qui lui est postérieur et l'air passe facilement. Mais c'est couché sur le dos que le voile s'applique le mieux sur le pharynx. C'est là la position habituelle du ronfleur sévère, et c'est dans cette position que les risques asphyxiques sont les plus grands. Le rétrécissement de la cavité buccale constitue le troisième élément responsable du ronflement. Ce rétrécissement est entraîné par l'obésité, l'obstruction nasale et le rétrognathisme.

Au niveau du pharynx, le ronflement traduit la vibration du voile du palais et des parois du pharynx sous l'action du flux aérien anormalement turbulent. Il est en rapport avec les caractéristiques anatomiques et mécaniques du pharynx et du mucus qui constitue une couche visqueuse superficielle des tissus du pharynx. Le mucus est un élément physiologique et anatomique d'importance car il constitue le milieu qui va interagir avec le spray (les actifs sont projetés et doivent agir à ce niveau). Il est donc particulièrement important de connaître précisément les caractéristiques physiques et physiologiques de ce milieu biologique.

Entre la cavité nasale et la trachée, toutes deux rigides et indéformables, l'air inspiratoire va traverser un espace déformable, élastique et plus ou moins rétréci. Dans ce rétrécissement vont apparaître des modifications de débit et de pression appelées phénomènes de Venturi, régis par les théorèmes de Bernouilli, à l'origine de la vibration des parois. Entre les fosses nasales et la trachée rigide, les parois de l'orohypopharynx sont molles, élastiques et rétrécies. Tout est donc réuni pour que se produise plusieurs fois par seconde le phénomène de Venturi, qui rend compte de la vibration du voile du palais lié au claquement de celui-ci contre les parois du pharynx. Cependant, à cause des réactions de défense provoquées par cette gêne inspiratoire se crée un cercle vicieux comportant une majoration de l'effort inspiratoire sous l'effet de cette obstruction - destinée à lutter contre l'asphyxie que celui-ci entraîne -mais responsable à son tour d'une obstruction qui est d'autant plus rapide et plus importante que cet effort est puissant. Ce contrôle volontaire étant impossible lors du sommeil, un ronflement à partir du moment où il s'installe ne peut que s'aggraver jusqu'à ce que l'éveil ou le semi éveil, redonnant du tonus aux muscles de l'oropharynx, supprime le rétrécissement et ses effets nocifs.

Pour que la vibration se produise, il faut que successivement, et environ 30 fois par minute, le voile mou touche la paroi postérieure du pharynx, qu'il en soit repoussé par un courant d'air venu du nez, puis qu'une force quelconque le ramène contre le palais. Dans le ronflement délibéré, cette force est la contraction volontaire. Dans le ronflement nocturne passif en position couchée, cette force est celle de la pesanteur, et quand elle s'applique sur un voile volumineux et long, celui-ci tend spontanément à se coller sur le pharynx.35 Ronflement au niveau nasal

Les ronflements d'origine nasale sont la conséquence d'un rétrécissement des voies nasales. Plus les tissus se relâchent ou plus le passage de l'air est difficile, plus le risque de ronfler est grand. La respiration sonore peut être provoquée ou accentuée par des polypes dans les voies nasales (un polype est une excroissance longiligne qui se produit sur une muqueuse); une congestion des voies nasales (rhume, allergie, absorption d'alcool ou de tranquillisants) et le relâchement des tissus avec l'âge. La cavité nasale est bordée d'un épithélium pseudostratifié qui repose sur une membrane basale, le séparant des couches profondes sous-muqueuses. Les couches sous-muqueuses sont composées de mucus, de séromucus et de glandes séreuses. Le flux sanguin local est distribué aux cellules grâce à un réseau de petites artères, artérioles et anastomoses artério-veineuses. La capacitance vasculaire, formée des veines et les sinusoïdes caverneux, détermine le degré d'ouverture des voies nasales. La contraction ou la relaxation de cet ensemble de vaisseaux est sous l'influence du système sympathique. Les sinusoïdes caverneux sont plus denses que les cornets inférieurs et moyens, et sont composés de cellules musculaires lisses contrôlées par le système nerveux sympathique. Une perte du tonus sympathique, ou à un moindre degré une stimulation cholinergique provoque un engorgement de ce tissu sinusoïdal érectile. L'ouverture des voies nasales est contrôlée principalement par les modifications qui interviennent dans la capacitance des vaisseaux. La résistance des voies nasales supérieures et en effet responsable pour les deux tiers de la résistance totale à l'air des voies respiratoires. Les sites prédominants de l'obstruction nasale au passage de l'air sont le vestibule nasal, les valves nasales et les cornets nasaux.

Il existe des variations physiologiques de l'ouverture des voies nasales, connues sous le nom de "cycle nasal". Ces variations ont pour conséquence principale des modifications de capacitance des vaisseaux sanguins situés dans les cornets inférieurs et moyens. Ces modifications alternent d'une cavité nasale à l'autre environ toutes les deux à quatre heures chez 70% des personnes. La posture intervient également dans la régulation du degré de congestion vasculaire. L'obstruction nasale augmente de manière bilatérale lorsque le sujet se place en position supine, et augmente de manière unilatérale selon la position latérale du sujet.

L'origine nasale du ronflement est une augmentation des résistances au passage de l'air des voies nasales supérieures, et plus précisément au niveau de la valve nasale et des cornets inférieurs et moyens. Ceci est la résultante d'un mécanisme physiopathologique de congestion, se traduisant par une dilatation des vaisseaux sanguins, une sécrétion de mucus augmentée, une dilatation des structures musculaires lisses. La résistance au passage de l'air augmente et modifie les flux d'air provoquant des vibrations des tissus mous du palais et de la luette.

### Chez les gros ronfleurs

Il semble que chez les gros ronfleurs, les modifications physiologiques retrouvées au niveau des voies nasales et au niveau du pharynx produisent des ronflements caractéristiques, que l'on ne retrouve pas chez les ronfleurs dont l'origine du ronflement est soit nasale, soit au niveau du pharynx. Les ronflements des gros ronfleurs se caractérisent en effet par une fréquence et une intensité particulières.35 Les ronflements chez un ronfleur "standard" ont une fréquence comprise entre 200 et 2000 Hz, avec des niveaux de décibels allant jusqu'à 70 dB. La fréquence varie en fonction de l'origine des ronflements [Perez-Padilla JR, Slawinski E, Difrancesco LM, Feige RR, Remmers JE, Whitelaw WA. Characteristics of the snoring noise in patients with and without occlusive sleep apnea. Am Rev Respir Dis. 1993 Mar;147(3):635-44]. Les ronflements des gros ronfleurs peuvent être assez puissants en intensité. La moyenne des sujets a un ronflement compris entre 40 et 50 dB, avec des sujets allant jusqu'à 80-90 dB et parfois même 105 dB. Il semble que le ronflement d'origine nasale ait une intensité plus faible que le ronflement d'origine buccale. Chez certains gros ronfleurs, l'intensité du ronflement est telle qu'elle entraîne des problèmes d'audition chez leurs partenaires.

Des études scientifiques ont été menées de façon à préciser plus en détail, chez les gros ronfleurs, s'il existait des modifications de la cavité du pharynx, des variations d'intensité et de passage des flux d'air inspirés et expirés. Grâce à ces données, la société Persee Médica a pu mettre en évidence quelle était la relation entre les causes nasales et buccales du ronflement chez les gros ronfleurs.

Green et collègues montrent dans une étude sur 18 volontaires, répartis en deux groupes selon qu'ils ronflaient ou non, que le volume de la cavité du pharynx n'est pas différent d'un groupe à l'autre [Green DE, Block AJ, Collop NA, Hellard DW. Pharyngeal volume in asymptomatic snorers compared with nonsnoring volunteers. Chest. 1991 Jan;99(1):49-53]. Cet élément n'est donc pas un élément discriminant chez les gros ronfleurs.

L'intensité et la fréquence du ronflement sont des caractéristiques discriminantes. En effet, les ronflements d'origine nasale et buccale ont des intensités différentes et des fréquences différentes. Chez les gros ronfleurs, il y a superposition des différentes intensités pour une même fréquence et addition d'intensités à des fréquences différentes, ce qui donne cet aspect de bruit diffus sur les spectres d'enregistrement. Stanescu et son équipe montrent que les gros ronfleurs présentent des problèmes de limitation de l'air inspiré et expiré [Stanescu D, Kostianev S, Sanna A, Liistro G, Veriter C. Expiratory flow limitation during sleep in heavy snorers and obstructive sleep apnoea patients. Eur Respir J. 1996 Oct;9(10):2116-21].

L'ensemble des modifications et perturbations qui se mettent en place chez les gros ronfleurs entraîne une modification des flux d'air aux niveaux buccal et nasal produisant un ronflement très important, très caractéristique à la fois en termes de fréquence, d'intensité et du profil du spectre d'enregistrement. Les gros ronfleurs présentent simultanément des modifications des flux d'air au niveau du nez ainsi qu'une vibration du voile du palais. Ces phénomènes ne sont pas indépendants les uns des autres mais, au contraire, fortement corrélés.

La vibration du voile du palais se fait principalement sous l'influence des flux d'air en provenance des voies nasales (souvent augmentés suite à une gêne respiratoire).

Cette vibration est elle-même entretenue par une diminution de la tonicité des muscles du pharynx et de la luette et produit une inflammation locale qui à son tour augmente l'atonie. Un premier "cercle vicieux" se constitue et entretien ainsi le phénomène de ronflement. De même, la vibration du voile du palais engendre des perturbations du passage de l'air lors de l'inspiration et de l'expiration et par voie de conséquence une majoration de l'effort respiratoire au niveau des voies nasales. Ceci entretient une inflammation localisé, une congestion et renforce ainsi le phénomène. Est ainsi présent un second "cercle vicieux" qui renforce les phénomènes à l'origine de ronflements chez les gros ronfleurs. Cette imbrication de plusieurs déséquilibres produit des ronflements de très forte intensité et de fréquence caractéristique chez les gros ronfleurs [voir figure en annexe].

### L'art antérieur

Les produits commercialisés contre le ronflement sont des préparations à base d'huiles permettant de lubrifier les muqueuses du pharynx ou nasales. Ces produits sont souvent perçus comme peu efficaces par une grande majorité de consommateurs. En effet, il est souvent mentionné que ces produits agissent rapidement mais perdent de leur efficacité dans la première heure du sommeil, peu de temps après l'application. Cette constatation s'explique par le fait que ces produits ne restent pas en place sur leur site d'action et sont rapidement éliminés vers l'arrière-gorge (sprays buccaux). En effet, la vélocité du mucus et l'influence du flux salivaire constant provoquent une élimination des produits huileux avec pour principale conséquence une faible durée d'action.

Certains produits contiennent des huiles essentielles. Les huiles essentielles utilisées dans ces préparations ne sont, pour certaines d'entres-elles, pas dénuées de toxicité. Certaines huiles essentielles font en effet l'objet de restrictions d'emploi et de mises en garde suite à la mise en évidence de leur toxicité signalée par les enquêtes de pharmacovigilance. De plus, la pulvérisation de préparations huileuses au niveau du pharynx et nasal est associée à des risques de pneumopathies huileuses, en raison du passage de gouttelettes lipidiques au niveau des poumons. Devant l'apparition en France de quelques cas de pneumopathies huileuses suite à l'administration de produits antironflement contenant des huiles, les Autorités de Santé Françaises (AFSSAPS) ont fait part aux fabricants des produits incriminés de leur préoccupation et ont demandé des données complémentaires sur la sécurité de ces produits. Le risque de passage au niveau pulmonaire des huiles contenues dans ces préparations représente en effet un risque certain pour la sécurité du consommateur. Enfin, il n'existe pas de produit permettant une action simultanée sur l'origine nasale et buccale du ronflement.

### Description de l'invention

Les travaux de recherche réalisés par la Demanderesse ont permis de mettre au point une nouvelle stratégie de traitement et de prévention des ronflements particulièrement adaptée aux gros ronfleurs. En effet, l'interaction entre un ronflement nasal et un ronflement dont l'origine est le pharynx (luette) et les imbrications entre ces deux phénomènes ont pour conséquence la production d'un niveau sonore de ronflements plus important.

Dans le cas de ronflements chez les gros ronfleurs, ceci implique nécessairement la mise en place d'un traitement simultané de ces deux cibles différentes.

Ce but est atteint selon l'invention grâce à l'utilisation d'une composition nasale et d'une composition buccale pour la préparation d'une composition combinée pour une utilisation simultanée, séquentielle ou séparée pour le traitement ou la prévention des ronflements chez un sujet, plus particulièrement chez un gros ronfleur dans laquelle la composition nasale comprend une combinaison d' un extrait de Ruscus aculeatus et de rutine, et;
- une première substance ou combinaison de substances assurant la diminution de la résistance de l'air au niveau de la valve nasale et des cornets moyens et inférieurs par la formation d'un "tapis de glissement" et la lubrification des tissus des voies nasales;
- une deuxième substance ou combinaison de substances présentant des propriétés de bio-adhésivité et permettant de maintenir au niveau de leur site d'action les première(s) et deuxième(s) substances.

Cette association pouvant être présentée groupée dans la même offre, ou bien séparée par deux offres individuelles regroupées sous une quelconque forme. La composition nasale se présente de préférence sous la forme d'un spray nasal comprenant : une substance ou combinaison de substances assurant une action décongestionnante afin d'augmenter la lumière nasale par une action de type sympathique sur les muscles lisses, une action de vasoconstriction des vaisseaux sanguins et une action anti-inflammatoire locale afin de diminuer la sécrétion exagérée de mucus et de substances vasodilatatrices; - une première substance ou combinaison de substances assurant la diminution de la résistance de l'air au niveau de la valve nasale et des cornets moyens et inférieurs par la formation d'un "tapis de glissement" et la lubrification des tissus des voies nasales; - une deuxième substance ou combinaison de substances présentant des propriétés de bio-adhésivité et permettant de maintenir au niveau de leur site d'action les première(s) et deuxième(s) substances.

La deuxième substance ou combinaison de substances est choisie dans le groupe comprenant : l'eau de mer, la glycérine et la phosphatidyleholine. La deuxième substance ou combinaison de substances est choisie dans le groupe des carraghénanes.

L'extrait de Ruscus aculeatus vise à provoquer une action de vasoconstriction des vaisseaux sanguins sous la forme d'une action de type adrénergique sur les muscles lisses des parois des vaisseaux et à augmenter la lumière nasale et par voie de conséquence de diminuer les résistances au passage de l'air dans les voies nasales. Au cours d'un essai publié en 2000 et portant sur 148 sujets suivis durant 12 semaines, un extrait de Ruscus aculeatus s'est révélé plus efficace qu'un placebo pour soulager les symptômes de l'insuffisance veineuse chronique en provoquant une vasoconstriction des parois veineuses [Lucker P, Jost V, Wolna P, et al. Efficacy and safety of ruscus extract compared to placebo in patients suffering from chronic venous insufficiency. Phytomedicine. 2000;7(suppl 2):P-155].

La rutine permet d'initier une action anti- inflammatoire locale afin de diminuer la sécrétion exagérée de mucus et de substances vasodilatatrices, de façon à diminuer l'oedème, la vasodilatation consécutive à l'inflammation et le relâchement des structures tissulaires des muscles lisses et à permettre, par voie de conséquence, une augmentation de la lumière nasale. La rutine fait partie de la grande famille des bioflavonoïdes. Elle est chimiquement très proche de la quercétine (un autre flavonoïde), et elle possède des propriétés antioxydantes, anti-inflammatoires, vasoprotectrices (protection des vaisseaux sanguins) et anti-thrombotiques (protection contre la formation de caillots sanguins). La rutine est présente dans plusieurs plantes médicinales, dont l'eucalyptus, l'aubépine, le ginkgo biloba et le millepertuis. Des expérimentations ont été menées chez l'animal. 35 Elles montent que l'activité anti-inflammatoire de la rutine est plus prononcée lors d'une inflammation chronique que celles d'autres composants tels que la quercétine et l'hespéridine [Guardia T, Rotelli AE, Juarez AO, Pelzer LE. Ariti-inflammatory properties of plant flavonoids. Effects of rotin, quercetin and hesperidin on adjuvant arthritis in rat. Farmaco. 2001 Sep;56(9):683-7].

La glycérine et la phosphatidylcholine assurent des fonctions de lubrification. L'eau de mer permet d'obtenir un tapis de glissement facilitant le passage de l'air au niveau des voies nasales en diminuant les mécanismes de résistance. L'eau de mer possède de nombreuses vertus thérapeutiques et principalement par voie locale interne, tout particulièrement dans les affections nasales. L'instillation locale de solution physiologique d'eau de mer permet une diminution de l'inflammation de la muqueuse nasale et l'obtention d'un tapis de glissement. L'eau de mer contient de nombreux sels minéraux et oligoéléments qui permettent de restaurer les fonctions ciliées de la muqueuse.

La composition nasale de l'invention est remarquable en ce qu'elle offre des propriétés de lubrification des muqueuses nasales et du pharynx sur une longue période grâce aux carraghénanes, qui possèdent des propriétés bio-adhésives sur les muqueuses.

L'ensemble de ces substances permet au spray nasal de diminuer les résistances de l'air au niveau des voies nasales. Cette diminution provoque en conséquence une diminution des turbulences des flux d'air au niveau du pharynx provoquées par l'augmentation de la respiration buccale.

Dans la composition nasale de l'invention, et au titre de la première substance ou combinaison de substances : - l'extrait de ruscus, s'il est présent, représente de l'ordre de 0,01 à 5 % ou de l'ordre de 0,03 à 0,07 % et de préférence de l'ordre de 0,5% en poids de la composition, - la rutine, si elle est présente, représente de l'ordre de 0,01 à 0,05 % et de préférence de l'ordre de 0,15% en poids de la composition, l'une au moins des substances ci-dessus étant présente dans la composition.

Dans la composition nasale de l'invention, et au titre de la deuxième substance ou combinaison de substances : l'eau--•de mer, si elle est présente,-représente de l'ordre de 0,1 à 10 % ou de l'ordre 0,5 à 5 % et de préférence de l'ordre de 1 % en poids de là composition, - la glycérine, si elle est présente, représente de l'ordre de 1 à 10 % ou de l'ordre de 1 à 4 % et de préférence de l'ordre de 2,65% en poids de la composition, -la phosphatidylcholine si elle est présente, représente de l'ordre de 0,5 à 10 % ou de l'ordre de 0,5 à 2,5 % et de préférence de l'ordre de 1,66 % en poids de la composition, l'une au moins des substances ci-dessus étant présente dans la composition. Tout particulièrement, la composition comprend un rapport pondéral deuxième substance / première substance allant de 20 à 100, notamment de 30 à 80, voire de 40 à 70.

Dans la composition nasale de l'invention, et au titre de la deuxième substance ou combinaison de substances, les carraghénanes représentent de l'ordre de 0,5 à 2 % et de préférence de l'ordre de 1 % de la composition. La composition peut comprendre un rapport pondéral deuxième substance /substance allant de 1 à 50, notamment de 2 à 20, voire 5 à 15.

La composition peut comprendre un rapport pondéral première substance / deuxième substance allant de 1 à 50, notamment de 2 à 20, voire 3 à 10.

L'invention concerne l'association de la composition nasale ci-dessus avec une composition buccale se présentant sous la forme d'un spray buccal. Ainsi, l'invention se rapporte à une méthode pour traiter ou prévenir les ronflements d'un sujet consistant en ce que ledit sujet reçoit une association d'une composition nasale ci-dessus avec une composition buccale se présentant sous la forme d'un spray buccal. Ainsi, l'invention se rapporte à l'utilisation d'une composition nasale telle que définie précédemment pour le traitement ou la prévention des ronflements chez un sujet, plus particulièrement chez un gros ronfleur, ladite composition nasale étant reçue par ledit sujet en association avec une composition buccale contre le ronflement.

La composition buccale comprend au moins une substance lubrifiante et au moins une substance bio-adhésive apte à faire adhérer ladite substance lubrifiante aux cellules mucocilliaires situées au niveau du pharynx. La substance bio-adhésive est choisie dans le groupe comprenant : des polysaccharides, des dérivés de la cellulose, des dérivés acryliques ou des dérivés de protéines. De préférence, la substance bio-adhésive est un polysaccharide appartenant à la famille des carraghénanes.

La composition buccale comprend de 0,5 à 20% environ de substance bio-adhésive, et en particulier de carraghénanes. Cette composition contient de 1 à 5% environ, et de préférence de 1,5 à 3% environ, de substance bioadhésive, et en particulier de carraghénanes. Elle peut aussi contenir de 5 à 20% environ, et de 10 à 15% environ, de substance bio-adhésive, et en particulier de carraghénanes.

Dans la composition buccale, la substance lubrifiante est un agent tensioactif présentant une partie polaire et une partie hydrophobe. La partie polaire de l'agent tensioactif est choisie dans le groupe comprenant : du glucose, du sucrose, du lactose, du glycérol, du xylose, des peptides, des acides aminés, des nucléotides. La partie hydrophobe est choisie dans le groupe comprenant : des acides gras, un alcool gras, une amine grasse, des esters, des glycérides, des phospholipides. La partie hydrophobe est constituée de phospholipides, par exemple de phosphatidylcholine.

Cette composition buccale contient de 0,5 à 20% environ de substance lubrifiante, et en particulier de phosphatidylcholine. Elle peut contenir de préférence de 2 à 10% environ de substance lubrifiante, et en particulier de phosphatidylcholine.

Cette composition buccale comprend en outre un ou plusieurs agent(s) anti-inflammatoire(s) et/ou tonique(s) des muqueuses et des tissus du pharynx. L'agent tonique des muqueuses et des tissus du pharynx est du cynorrhodon. La composition buccale contient entre 0,01 et 5% d'agent tonique, et en particulier de cynorrhodon. La composition buccale se présente sous la forme d'un aérosol plus particulièrement d'une mousse. Une composition buccale préférée contient : - de 1 à 5% environ, et de préférence de 3% environ, de carraghénanes ; - de 1 à 10% environ, et de préférence de 5% environ, de phosphatidylcholine ; - de 0,01 à 5% environ, et de préférence de 2% 35 environ, de cynorrhodon.

La composition du spray buccal permet de lubrifier les parois du pharynx (luette) et d'augmenter la tonicité des structures musculaires lisses tout au long de la nuit grâce à l'action bio-adhésive des carraghénanes. Le spray nasal permet de réduire la congestion nasale, de diminuer l'inflammation chronique et de lubrifier les parois pour faciliter le passage des flux d'air lors de l'inspiration et de l'expiration.

Les travaux de recherche réalisés par la Demanderesse lui ont permismettre en évidence une synergie entre carraghénanes et glycérine. En effet, les propriétés de bioadhésion des carraghénanes sont significativement augmentées par la glycérine.

L'invention propose la mise en oeuvre tant dans la composition nasale que dans la composition buccale de deux agents importants que sont les carraghénanes et la glycérine. Les carraghénanes sont utilisés pour leurs propriétés de bio-adhésion. La glycérine permet d'émulsifier et ainsi de disperser la phosphatidylcholine qui est un agent lubrifiant à caractère lipophile) dans la composition finale à caractère hydrophile). Les carraghénanes présentent des propriétés de bio- adhésion. La glycérine en revanche ne présente aucune propriété de bio-adhésion. La glycérine est un agent que l'on retrouve dans une multitude de préparations pharmaceutiques. Cet agent texturant n'est pas décrit dans la littérature comme un agent bio-adhésif. La glycérine engendre dans la composition une augmentation modérée de la viscosité. Or de manière surprenante, la Demanderesse grâce à plusieurs expérimentations a découvert que la glycérine augmentait fortement la bio-adhésion des carraghénanes. En effet, la glycérine augmente les facteurs de bio-adhésivité de la préparation même lorsque ces derniers sont corrigés de l'influence de la viscosité. Ainsi en présence de glycérine, le facteur corrigé d'aptitude à l'adhésion est multiplié par 200 et le facteur de bio-adhésivité est augmenté de 100%.

Comme cela a été mentionné précédemment, la glycérine ne présente pas de propriétés de bio-adhésion. Pourtant, ces expérimentations démontrent qu'elle joue un rôle de synergie avec les carraghénanes afin d'augmenter fortement leurs propriétés bio-adhésives.

Cette propriété s'applique également à d'autres agents bio-adhésifs quels qu'ils soient, et en particulier des agents tels que les carbopols et les autres dérivés de la cellulose. En effet, les carraghénanes présentent de nombreux groupements facilitant la formation de liaisons hydrogènes en présence d'eau ou de molécules possédant des groupements hydrophiles. Ces groupements sont des hydroxyles (-OH), présents sur les molécules de galactose, unité de base du polymère polysaccharidique que sont les carraghénanes. Ces propriétés structurelles présentent des similitudes avec de nombreux autres composés bio-adhésifs tels que les carbopols par exemple. Les carbopols sont des polymères réticulés de l'acide acrylique. En étudiant les similarités de structures entre carraghénanes et carbopols, il. est possible de conclure que ces substances et dérivés augmenteront les propriétés de bio-adhésion des carraghénanes. L'invention concerne encore un pack ou une trousse, ou encore un ensemble, comprenant les deux compositions précédentes de façon à associer sous une forme quelconque les deux modes d'action combinés, ainsi qu'une notice d'utilisation de façon à offrir au sujet atteint de ronflement un traitement basé sur leur action conjuguée permettant de s'attaquer, de manière simultanée aux deux causes du bruit provoqué chez les gros ronfleurs. Les deux produits qui composent cette trousse ou ce pack peuvent être utilisé dans un ordre précis ou au contraire de manière non ordonnée. Les compositions composant ce pack peuvent être conditionnées dans un contenant comprenant plusieurs enceintes, ou dans des contenants séparés. Les contenants pouvant être conditionnés dans un seul emballage. Le pack peut se présenter sous une forme duo, mix ou offre jumelée. Ainsi, on entend aussi par pack, tout conditionnement, où les deux compositions sont associées, constitué de carton, papier, plastique transparent de couleur, éventuellement de couleur, de taille, même de matière, différentes, etc.

Cette combinaison de compositions permet pour la première fois de traiter les interactions entre les modifications physiologiques nasales et pharyngées à l'origine du ronflement chez les gros ronfleurs. En effet, il y a mise en place de plusieurs "cercles vicieux" qui s'installent progressivement, renforcés par des épisodes d'obstruction des voies respiratoires supérieures qui induisent des modifications des flux d'air. Seule la combinaison des solutions techniques apportées par l'invention permet d'agir simultanément sur les causes du ronflement afin de diminuer significativement, voire de supprimer, le bruit des ronflements chez les gros ronfleurs.

Dans la présente invention, sauf indications contraires, les % sont des % en poids par rapport au poids total de la composition. D'autres caractéristiques de l'invention apparaîtront des exemples de compositions nasales et buccales qui 30 suivent.

Composition nasale Gros Ronfleurs (spray nez) [qsp: quantité suffisante pour ...]

| Ingrédients | Dosage dans le produit fini | Fourchette |
|---|---|---|
| Carraghénanes | 1% | 0,1 - 10% |
| Phosphatidylcholine | 1,66% | 0,5 - 10% |
| Eau de mer | 1% | 0,1 - 10% |
| Ruscus (extrait de Ruscus aculeatus) | 0,5% | 0,01 - 5% |
| Rutine | 0,15% | 0,01 - 5% |
| Glycérine | 2,65% | 1 - 10% |
| Additifs, conservateurs et arômes | / | / |
| Eau | qsp 100% | qsp 100% |

Composition buccale (spray gorge) [qsp: quantité suffisante pour...]

| Ingrédients | Dosage dans le produit fini | Fourchette |
|---|---|---|
| Carraghénanes | 3% | 1 - 20% |
| Phosphatidylcholine | 5% | 1 - 10% |
| Reine des prés (extrait de Filipendula ulmaria) | 3% | 1 - 5% |
| Cynorrhodon (extrait de Rosa canina) | 0,3% | 0,01 - 5% |
| Glycérine | 8% | 1 - 20% |
| Additifs, conservateurs et arômes | / | / |
| Eau | qsp 100% | qsp 100% |

D'autres avantages et caractéristiques de l'invention apparaîtront des exemples qui suivent dans lesquels il sera fait référence aux figures en annexe dans lesquels : - la figure 1 représente les courbes des enregistrements et courbe de référence des personnes ronfleurs ne prenant aucun produit, - la figure 2 représente les courbes des 5 enregistrements et courbe de référence des personnes ronfleurs prenant SILENCE Gorge, - la figure 3 représente les courbes des enregistrements et courbe de référence des personnes ronfleurs prenant SILENCE Gros Ronfleurs, 10 - la figure 4 représente les courbes des enregistrements et courbe de référence des personnes ronfleurs, - la figure 5 représente la diminution de la durée des ronflements en fonction de l'utilisation ou non du 15 produit.

### I Description des compositions.

Les compositions suivantes ont été utilisées dans les expériences rapportées ci-après. - Silence Gros Ronfleurs spray buccal : Agents lubrifiants (dont la phosphatidylcholine), agents bioadhésifs et renforçant la :bio-adhésion (carraghénanes et glycérine), agents tonifiants les muqueuses, excipients technologiques. - Silence Gros Ronfleurs spray nasal : Agents favorisant le passage de l'air (lubrifiant, vasoconstricteur, décongestionnant), agents bio-adhésifs et renforçant la bio-adhésion (carraghénanes et glycérine), excipients technologiques. -DouceNuit spray buccal : Agents lubrifiants, excipients technologiques.

### II - Méthodologie

1) Étude du bruit de fond d'une chambre. L'objectif était d'avoir une référence permettant d'évaluer le niveau sonore d'une chambre occupée par un couple de non ronfleur et de considérer ce bruit comme un bruit de fond, c'est-à-dire un bruit inhérent à la respiration et aux mouvements de personnes endormies. Il faut remarquer que ce bruit de fond est extrêmement sensible à l'environnement, selon la situation de l'appartement ou de la maison en milieu urbain ou rural. Dans cette enquête, un certain nombre de conditions préalables devaient être remplies, et notamment une situation de la chambre "au calme", une chambre protégée par du double vitrage, dont :La porte est restée fermée tout au long de la nuit. De plus, le regroupement des mesures par le calcul de moyennes permet d'exclure les perturbations provoquées par des influences externes temporaires (passage d'une voiture dans la rue), qui constituent une élévation importante de décibels sur une courte période et interviennent de ce fait, très peu dans le calcul de la moyenne. 2) Etude du bruit généré par couple de un ou deux ronfleurs. L'objectif était d'évaluer le bruit généré par le ronfleur lorsque celui-ci n'utilise aucun produit. Le nouveau produit Silence devant permettre une réduction du bruit significatif comparativement à ce niveau sonore. Dans ce cas précis, les deux personnes partageant la chambre prenaient ou non l'un des produits, selon le protocole opératoire. 3) Étude du bruit généré un couple d'un ou deux ronfleurs utilisant Silence Gorge. L'objectif était d'évaluer les gains de niveau de bruit obtenus par le produit SILENCE Gorge. 4) Étude du bruit généré par un couple de un ou deux ronfleurs utilisant le produit Silence Gorge et Silence Gros Ronfleurs. L'objectif final était de mesurer les performances obtenues par les différents produits et de les comparer. L'étude a également analysé un produit présent sur le marché est dénommé DouceNuit. Ce produit est commercialisé sous la forme d'un spray buccal, d'un spray nasal et de bandelettes à appliquer sur la langue.

Les enregistrements ont été réalisés tout au long de la nuit, de 23 heures à 5 heures du matin. Seule la période de 1 heure à 3 heures du matin a été retenue pour l'analyse du son. Le logiciel de l'appareil permet en effet de constituer à partir d'un enregistrement un rapport d'analyse comprenant les données suivantes moyenne de l'enregistrement et médiane, période de ronflement (> 55 dB), durée de l'enregistrement et durée d'analyse de l'intervalle, enfin, pour chaque niveau d'intensité allant de 99 dB à 36 dB (par diminution de 3 dB), le pourcentage de l'intervalle supérieur à cette valeur d'intensité. Les produits utilisés ont été Silence Gorge et Silence Gros Ronfleurs.

### III - Résultats

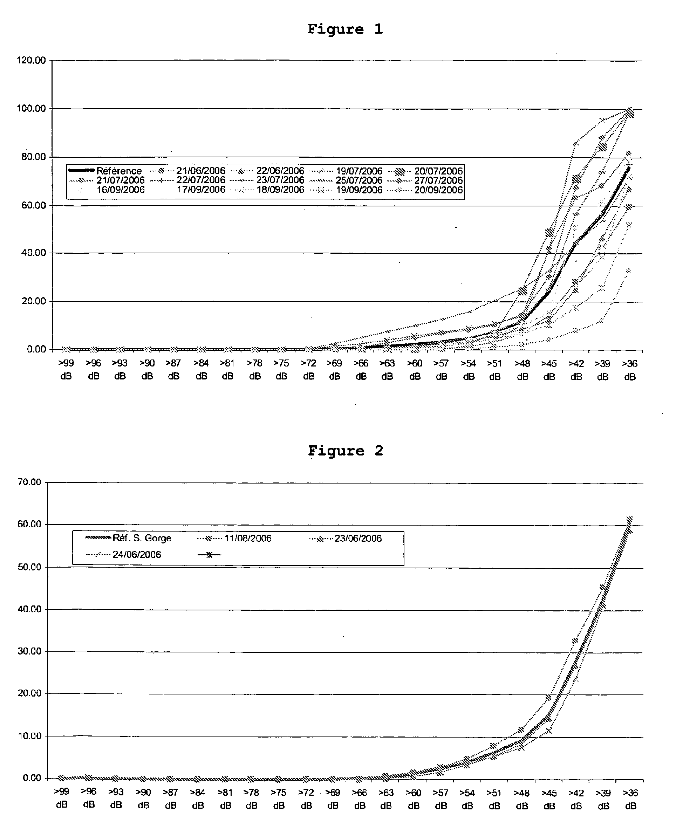

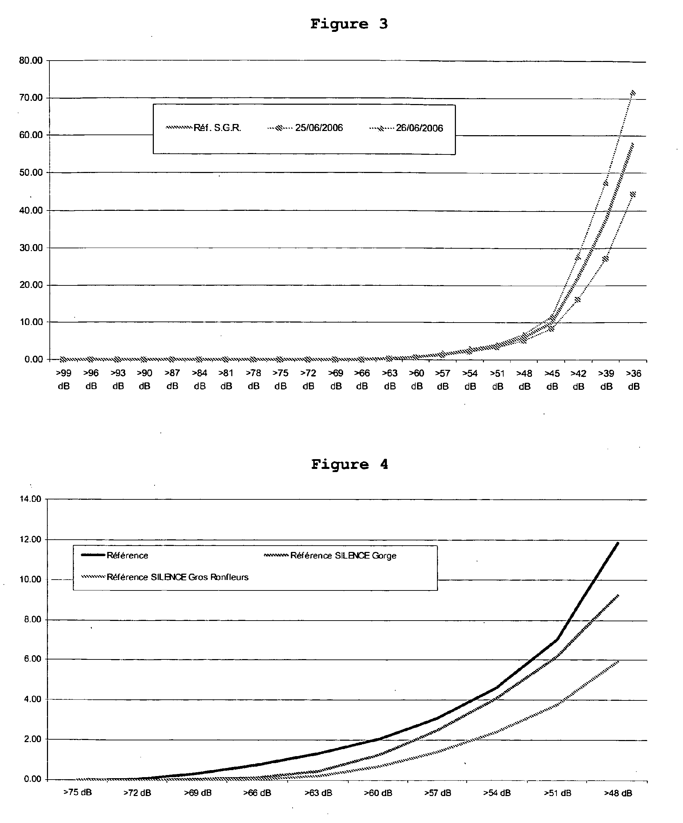

Les courbes présentées dans les figures en annexe indiquent en abscisse les différents niveaux de mesure (par ex. >45 dB, >48 dB...) et l'ordonnée indique le pourcentage de l'intervalle mesuré pour chaque niveau de mesure. Les enregistrements montrent que la moyenne en intensité de niveau sonore d'une pièce contenant deux personnes non ronfleurs indique une valeur comprise entre 42 dB et 45 dB. Chez un couple de ronfleurs, le découpage selon l'intensité des différentes nuits enregistrées permet d'établir une courbe de référence. L'objectif de cette courbe de référence est de pouvoir réaliser une comparaison avec des courbes similaires lors de l'utilisation de Silence Gorge et de Silence Gros Ronfleurs. La courbe de référence est constituée à partir des moyennes des valeurs considérées pour chaque niveau de mesure. La figure 1 présente les différentes courbes d'enregistrement ainsi que la courbe de référence. Les courbes d'enregistrement ainsi que la courbe de référence des personnes ronfleurs prenant Silence Gorge et Silence Gros Ronfleurs sont respectivement présentées aux figures 2 et 3.

Les résultats obtenus par l'appareil sont exploitables et permettent de réaliser des courbes à partir des relevés des niveaux de décibels tout au long de la nuit et sur un intervalle de deux heures. Les relevés ont été réalisés à une fréquence de 1.200 Hz. L'intervalle de deux heures est compris entre 1 heure et 3 heures du matin. Les enregistrements montrent que la moyenne en intensité de niveau sonore d'une pièce contenant deux personnes non ronfleurs indique une valeur comprise entre 42 dB et 45 dB. Cette valeur est supérieure à la valeur trouvée lors d'une expérimentation précédente (36 à 39 dB). Ceci s'explique par la localisation géographique de ce test, l'appartement étant situé en milieu urbain (plus bruyant qu'un milieu rural) bien que bénéficiant cependant des conditions requises préalablement à l'expérimentation (double vitrage...). Il faut également noter qu'avec une moyenne de 45 dB pour un environnement de personnes non ronfleurs, on se situe à la limite de l'audibilité décrite par les courbes de Fletcher et Munson. Cette valeur conditionne les analyses des courbes d'enregistrement de personnes prenant ou non le produit. De façon à éliminer dans l'analyse les bruits consécutifs au milieu ambiant et aux personnes présentes dans la pièce, il est convenu de choisir comme limite inférieure un niveau d'enregistrement de 48 dB. De plus, les enregistrements montrent qu'au-delà de 75 dB, il n'y a plus d'enregistrement de sons. Ainsi, la fourchette d'analyse est définie entre 75 et 48 dB. Les trois courbes de référence (aucun produit - Silence Gorge - Silence Gros Ronfleurs) ont été placées sur la même courbe (Figure 4).

On constate sur la figure 4 que sur un intervalle de deux heures (1 heure à 3 heures du matin), les ronflements (intensité supérieur à 48 dB) chez un couple de ronfleurs ne prenant aucun produit concernent 12% de l'intervalle, soit environ 15 minutes. Chez les personnes utilisant du SILENCE Gorge, la durée des ronflements ne représente plus que 9% (10 minutes), soit une baisse de 30%. Enfin, toujours par rapport à la courbe de référence, chez un couple utilisant SILENCE Gros Ronfleurs, cette durée est diminuée à 6% de l'intervalle, soit 7 minutes. Dans ce dernier cas, la diminution est de 50%. Par rapport à Silence Gorge, la diminution est de 22%.

Ces résultats montrent l'importante efficacité des produits Silence Gorge et Gros Ronfleurs. Ils confirment la nécessité d'utiliser Silence Gros Ronfleurs chez les personnes présentant des ronflements d'origine nasale et buccale et confirment l'augmentation d'efficacité par l'utilisation simultanée des deux produits. En effet, sans prise de produit, la durée des ronflements au cours d'une nuit peut atteindre chez certaines personnes plus de 2 heures.

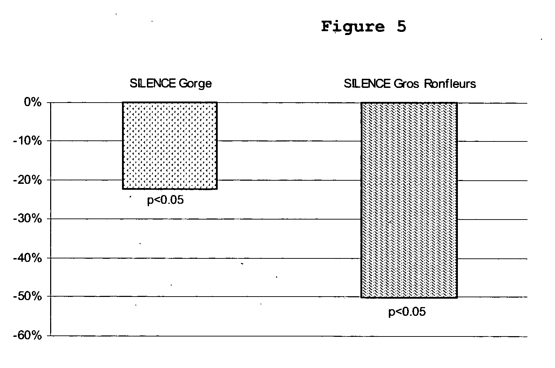

La figure 5 présente la diminution de la durée des ronflements en fonction de l'utilisation ou non du produit Silence Gorge ou Silence Gros Ronfleurs, par rapport à la courbe de référence (aucun produit).

Les tests de Student montrent que SILENCE Gorge et SILENCE Gros Ronfleurs présentent une diminution de l'intensité des ronflements statistiquement significative par rapport à la référence (p<0,05).

## Revendications

1. Utilisation non-thérapeutique d'une composition nasale et d'une composition buccale pour une utilisation simultanée, séquentielle ou séparée pour le traitement ou la prévention des ronflements chez un sujet, plus particulièrement chez un gros ronfleur, dans laquelle la composition nasale comprend une combinaison d' un extrait de Ruscus aculeatus et de rutine, et;
- une première substance ou combinaison de substances assurant la diminution de la résistance de l'air au niveau de la valve nasale et des cornets moyens et inférieurs par la formation d'un "tapis de glissement" et la lubrification des tissus des voies nasales;
- une deuxième substance ou combinaison de substances présentant des propriétés de bio-adhésivité et permettant de maintenir au niveau de leur site d'action les première(s) substances.

2. Utilisation selon la revendication 1, dans laquelle la deuxième substance ou combinaison de substances est choisie dans le groupe comprenant : l'eau de mer, la glycérine et la phosphatidylcholine, dans laquelle :
- l'eau de mer, si elle est présente, représente de l'ordre de 0,1 à 10 % et de préférence de l'ordre de 1 % en poids de la composition,
- la glycérine, si elle est présente, représente de l'ordre de 1 à 10 % et de préférence de l'ordre de 2,65% en poids de la composition,
- la phosphatidylcholine si elle est présente, représente de l'ordre de 0,5 à 10% et de préférence de l'ordre de 1,66 % en poids de la composition, l'une au moins des substances ci-dessus étant présente dans la composition.

3. Utilisation selon l'une quelconque des revendications 1 ou 2, dans laquelle la deuxième substance ou combinaison de substances est choisie dans le groupe des carraghénanes, dans laquelle les carraghénanes représentent de l'ordre de 0, 1 à 10 % et de préférence de l'ordre de 1 % de la composition nasale.

4. Utilisation selon l'une quelconque des revendications 2 à 3, dans laquelle:
- l'extrait de ruscus représente de l'ordre de 0,01 à 5 % et de préférence de l'ordre de 0,50% en poids de la composition,
- la rutine représente de l'ordre de 0,01 à 5 % et de préférence de l'ordre de 0,15% en poids de la composition.

5. Utilisation selon l'une quelconque des revendications précédentes, se présentant sous la forme d'un spray nasal.

6. Utilisation selon l'une quelconque des revendications précédentes, dans laquelle la composition buccale contre le ronflement comprend au moins une substance lubrifiante et au moins une substance bioadhésive apte à faire adhérer ladite substance lubrifiante aux cellules mucocilliaires situées au niveau du pharynx.

7. Utilisation selon la revendication 6, dans laquelle la substance bio-adhésive est choisie dans le groupe comprenant : des polysaccharides, des dérivés de la cellulose, des dérivés acryliques ou des dérivés de protéines.

8. Utilisation selon la revendication 7, dans laquelle la substance bio-adhésive est un polysaccharide appartenant à la famille des carraghénanes.

9. Utilisation selon l'une quelconque des revendications 6 à 8, dans laquelle la composition buccale contient de 0,5 à 20% environ de substance bio-adhésive, et en particulier de carraghénanes; dans laquelle la composition buccale contient de 0,5 à 20% environ de substance lubrifiante, de préférence de 2 à 10% environ, et en particulier de phosphatidylcholine.

10. Utilisation selon l'une quelconque des revendications 6 à 9, **caractérisée en ce que** la substance lubrifiante est un agent tensioactif présentant une partie polaire et une partie hydrophobe, dans laquelle la partie polaire de l'agent tensioactif est choisie dans le groupe comprenant : du glucose, du sucrose, du lactose, du glycérol, du xylose, des peptides, des acides aminés, des nucléotides, et dans laquelle la partie hydrophobe est choisie dans le groupe comprenant : des acides gras, un alcool gras, une amine grasse, des esters, des glycérides, des phospholipides.

11. Utilisation selon l'une quelconque des revendications 6 à 10, **caractérisée en ce que** la composition buccale comprend en outre un ou plusieurs agent(s) anti-inflammatoire(s) et/ou tonique(s) des muqueuses et des tissus du pharynx.

12. Utilisation selon la revendication 11, **caractérisée en ce que** l'agent tonique des muqueuses et des tissus du pharynx est du cynorrhodon.

13. Utilisation selon la revendication 11 ou la revendication 12, dans laquelle la composition buccale contient entre 0,5 et 5% d'agent tonique, et en particulier de cynorrhodon.

14. Utilisation selon l'une quelconque des revendications 6 à 13, **caractérisée en ce que** la composition buccale se présente sous la forme d'une mousse.

15. Pack comprenant au moins une composition nasale comme définie dans l'une quelconque des revendications 1 à 5 et au moins une composition buccale comme définie dans l'une quelconque des revendications 6 à 14.

## Patentansprüche

1. Nicht-therapeutische Verwendung einer nasalen und einer bukkalen Zusammensetzung für eine gleichzeitige, aufeinander folgende oder getrennte Verwendung zur Behandlung oder Vorbeugung von Schnarchen bei einem Individuum, insbesondere bei einem starken Schnarcher, wobei die nasale Zusammensetzung eine Kombination eines Extraktes von Ruscus aculeatus und Rutin umfasst, und
- eine erste Substanz oder Kombination von Substanzen, die die Verringerung des Luftwiderstands an der Nasenklappe sowie im Sinusgang und im Atemgang durch Bildung eines "Gleitteppichs" und die Befeuchtung der Gewebe der Nasengänge sicherstellt,
- eine zweite Substanz oder Kombination von Substanzen, die bioadhäsive Eigenschaften aufweist und es gestattet, die erste(n) Substanz(en) an deren Wirkungsort zu halten.

2. Verwendung nach Anspruch 1, wobei die zweite Substanz oder Kombination von Substanzen aus der Gruppe ausgewählt wird, die Folgende umfasst: Meerwasser, Glycerin und Phosphatidylcholin, wobei
- Meerwasser, falls vorhanden, in der Größenordnung von 0,1 bis 10 Gew.-% und vorzugsweise in der Größenordnung von 1 Gew.-%, bezogen auf das Gewicht der Zusammensetzung, ausmacht,
- Glycerin, falls vorhanden, in der Größenordnung von 1 bis 10 Gew.-% und vorzugsweise in der Größenordnung von 2,65 Gew.-%, bezogen auf das Gewicht der Zusammensetzung, ausmacht,
- Phosphatidylcholin, falls vorhanden, in der Größenordnung von 0,5 bis 10 Gew.-% und vorzugsweise in der Größenordnung von 1,66 Gew.-%, bezogen auf das Gewicht der Zusammensetzung, ausmacht, wobei mindestens eine der vorstehenden Substanzen in der Zusammensetzung vorhanden ist.

3. Verwendung nach einem der Ansprüche 1 oder 2, wobei die zweite Substanz oder Kombination von Substanzen aus der Gruppe der Carrageene ausgewählt wird, wobei die Carrageene in der Größenordnung von 0,1 bis 10 Gew.-% und vorzugsweise in der Größenordnung von 1 Gew.-%, bezogen auf das Gewicht der nasalen Zusammensetzung, ausmachen.

4. Verwendung nach einem der Ansprüche 2 bis 3, wobei:
- der Ruscus-Extrakt in der Größenordnung von 0,01 bis 5 Gew.-% und vorzugsweise in der Größenordnung von 0,50 Gew.-%, bezogen auf das Gewicht der Zusammensetzung, ausmacht,
- Rutin in der Größenordnung von 0,01 bis 5 Gew.-% und vorzugsweise in der Größenordnung von 0,15 Gew.-%, bezogen auf das Gewicht der Zusammensetzung, ausmacht.

5. Verwendung nach einem der vorhergehenden Ansprüche, die in Form eines Nasensprays dargereicht wird.

6. Verwendung nach einem der vorhergehenden Ansprüche, wobei die bukkale Zusammensetzung gegen Schnarchen mindestens eine Gleitsubstanz und mindestens eine bioadhäsive Substanz umfasst, die dazu geeignet ist, die Gleitsubstanz an den mukoziliären Zellen in der Rachenhöhle haften zu lassen.

7. Verwendung nach Anspruch 6, wobei die bioadhäsive Substanz aus folgender Gruppe ausgewählt ist:
Polysacchariden, Cellulosederivaten, Acrylderivaten oder Proteinderivaten.

8. Verwendung nach Anspruch 7, wobei die bioadhäsive Substanz ein Polysaccharid aus der Familie der Carrageene ist.

9. Verwendung nach einem der Ansprüche 6 bis 8, wobei die bukkale Zusammensetzung etwa 0,5 bis 20% an bioadhäsiver Substanz und insbesondere an Carrageenen enthält, wobei die bukkale Zusammensetzung etwa 0,5 bis 20% an Gleitsubstanz, vorzugsweise etwa 2 bis 10%, und insbesondere an Phosphatidylcholin enthält.

10. Verwendung nach einem der Ansprüche 6 bis 9, **dadurch gekennzeichnet, dass** die Gleitsubstanz ein oberflächenaktives Mittel mit einem polaren Teil und einem hydrophoben Teil ist, wobei der polare Teil des oberflächenaktiven Mittels aus der folgenden Gruppe ausgewählt ist: Glucose, Saccharose, Lactose, Glycerin, Xylose, Peptiden, Aminosäuren, Nukleotiden, und wobei der hydrophobe Teil aus folgender Gruppe ausgewählt ist: Fettsäuren, einem Fettalkohol, einem Fettamin, Estern, Glyceriden, Phospholipiden.

11. Verwendung nach einem der Ansprüche 6 bis 10, **dadurch gekennzeichnet, dass** die bukkale Zusammensetzung außerdem ein oder mehrere entzündungshemmende(s) und/oder stimulierende(s) Mittel für die Schleimhäute und die Gewebe der Rachenhöhle umfasst.

12. Verwendung nach Anspruch 11, **dadurch gekennzeichnet, dass** es sich bei dem stimulierenden Mittel für die Schleimhäute und die Gewebe der Rachenhöhle um Hagebutte handelt.

13. Verwendung nach Anspruch 11 oder Anspruch 12, wobei die bukkale Zusammensetzung zwischen 0,5 und 5% an stimulierendem Mittel und insbesondere an Hagebutte enthält.

14. Verwendung nach einem der Ansprüche 6 bis 13, **dadurch gekennzeichnet, dass** die bukkale Zusammensetzung in Form eines Schaums dargereicht wird.

15. Paket, das mindestens eine nasale Zusammensetzung nach einem der Ansprüche 1 bis 5 und mindestens eine bukkale Zusammensetzung nach einem der Ansprüche 6 bis 14 umfasst.

## Claims

1. Non-therapeutic use of a nasal composition and of a buccal composition for simultaneous, sequential or separate use for the treatment or prevention of snoring in a subject, more particularly in a heavy snorer, in which the nasal composition comprises a combination of a Ruscus aculeatus extract and rutin, and
- a first substance or combination of substances bringing about a reduction in air resistance in the region of the nasal valve and of the middle and inferior conchae by the formation of a "gliding mat" and the lubrication of the tissues of the nasal passages;
- a second substance or combination of substances exhibiting properties of bioadhesiveness and allowing the first substance(s) to be maintained in the region of their site of action.

2. Use according to Claim 1, in which the second substance or combination of substances is chosen from the group comprising: sea water, glycerine and phosphatidylcholine, in which:
- the sea water, if present, represents of the order of 0.1 to 10% and preferably of the order of 1% by weight of the composition,
- the glycerine, if present, represents of the order of 1 to 10% and preferably of the order of 2.65% by weight of the composition,
- the phosphatidylcholine, if present, represents of the order of 0.5 to 10% and preferably of the order of 1.66% by weight of the composition, at least one of the above substances being present in the composition.

3. Use according to either of Claims 1 and 2, in which the second substance or combination of substances is chosen from the group comprising carrageenans, in which the carrageenans represent of the order of 0.1 to 10% and preferably of the order of 1% of the nasal composition.

4. Use according to either of Claims 2 and 3, in which:
- the ruscus extract represents of the order of 0.01 to 5% and preferably of the order of 0.50% by weight of the composition,
- the rutin represents of the order of 0.01 to 5% and preferably of the order of 0.15% by weight of the composition.

5. Use according to any one of the preceding claims, being in the form of a nasal spray.

6. Use according to any one of the preceding claims, in which the buccal composition against snoring comprises at least one lubricating substance and at least one bioadhesive substance capable of causing the said lubricating substance to adhere to the mucociliary cells located in the region of the pharynx.

7. Use according to Claim 6, in which the bioadhesive substance is chosen from the group comprising:
polysaccharides, cellulose derivatives, acrylic derivatives or protein derivatives.

8. Use according to Claim 7, in which the bioadhesive substance is a polysaccharide belonging to the carrageenan family.

9. Use according to any one of Claims 6 to 8, in which the buccal composition contains from about 0.5 to 20% of bioadhesive substance, and in particular of carrageenans, and in which the buccal composition contains from about 0.5 to 20% of lubricating substance, preferably from about 2 to 10%, and in particular of phosphatidylcholine.

10. Use according to any one of Claims 6 to 9, **characterized in that** the lubricating substance is a surfactant having a polar part and a hydrophobic part, in which the polar part of the surfactant is chosen from the group comprising: glucose, sucrose, lactose, glycerol, xylose, peptides, amino acids, nucleotides, and in which the hydrophobic part is chosen from the group comprising: fatty acids, a fatty alcohol, a fatty amine, esters, glycerides, phospholipids.

11. Use according to any one of Claims 6 to 10, **characterized in that** the buccal composition additionally comprises one or more anti-inflammatory and/or tonic agent(s) for the mucous membranes and the tissues of the pharynx.

12. Use according to Claim 11, **characterized in that** the tonic agent for the mucous membranes and the tissues of the pharynx is rosehip.

13. Use according to Claim 11 or Claim 12, in which the buccal composition contains between 0.5 and 5% of tonic agent, and in particular of rosehip.

14. Use according to any one of Claims 6 to 13, **characterized in that** the buccal composition is provided in the form of a foam.

15. Pack comprising at least one nasal composition as defined in any one of Claims 1 to 5 and at least one buccal composition as defined in any one of Claims 6 to 14.
